# EUROPÄISCHE PATENTANMELDUNG

(11) **EP 1 813 911 A1**
(43) Veröffentlichungstag der Anmeldung: **01.08.2007**
(21) Anmeldenummer: 07001642.3
(22) Anmeldetag: 25.01.2007
(51) Int. Cl.: G01B 11/00

(54) **Positionsbestimmungssystem**

(30) Priorität: 25.01.2006 DE 102006003569
(71) Anmelder: AXIOS 3D Services GmbH, 26121 Oldenburg (DE)
(72) Erfinder: Broers, Holger, 26670 Uplengen (DE)
(74) Vertreter: Heinze, Ekkehard

(57) **Zusammenfassung**

Positionsbestimmungssystem zur Bestimmung der Lage eines Körpers oder Körperabschnittes im Raum, mit einem Markierungsinstrument, welches mindestens fünf strahlungserzeugende oder -reflektierende Markerelemente in im wesentlichen räumlich starrer Geometrie zueinander und zu einem Körperkontaktabschnitt des Instruments aufweist, zum mindestens temporären In-Kontakt-Bringen mit dem Körper oder Körperabschnitt, einer ortsfesten monoskopischen Bildaufnahmeeinrichtung einer Datenverarbeitungseinrichtung zur Verarbeitung des Bildes des Markierungsinstrumentes und zur Bestimmung einer Raumposition seines Körper-Kontaktabschnittes aufgrund einer Vermessung des Bildes bzw. der Bilder.

## Beschreibung

Die Erfindung betrifft ein Positionsbestimmungssystem zur Bestimmung der Lage eines Körpers oder Körperabschnittes im Raum nach dem Oberbegriff des Anspruchs 1. Derartige Positionsbestimmungssysteme werden insbesondere bei der Steuerung von Robotern (speziell Industrierobotern) sowie als Assistenzsysteme in der Chirurgie, speziell bei der Implantation von Gelenkimplantaten, eingesetzt.

In der Chirurgie werden derartige Systeme als optische Trackingsysteme zur präzisen dreidimensionalen Vermessung anatomischer Merkmale bzw. der Lage von Implantaten eingesetzt. Anhand der hiermit gewonnenen Referenzen können die Lage und Ausrichtung der Implantate optimiert werden. Hierbei haben sich optische Stereosysteme durchgesetzt, die zwei fest zueinander positionierte und kalibrierte Kameras umfassen und mit diesen ein räumliches Bild eines am zu vermessenden Körperteil oder Implantat angesetzten Markierungsinstrumentes gewinnen.

Mit bewährten fotogrammetrischen Verfahren, insbesondere unter Nutzung des Verfahrens des Vorwärtsschnittes, können die Raumpositionen der an einem solchen Markierungsinstrument vorgesehenen Markerelemente berechnet werden. Hieraus können dem Operateur Lage und Richtung von Extremitäten, von Schneidwerkzeugen während der Operation oder der Implantate während des Einsetzens angezeigt werden.

Diese Technologie ist etabliert und anerkannt, steht jedoch wegen der relativ hohen Kosten der technischen Ausrüstung nur Operateuren mit hohen Fallzahlen zur Verfügung.

Systeme der hier in Rede stehenden Art sind Gegenstand zahlreicher Schutzrechtsveröffentlichungen, die plastisch die Entwicklung hin zu immer flexibleren und leistungsfähigeren Systemen dokumentieren. Nur beispielhaft sei hier hingewiesen auf die EP 0 829 701 B1, die EP 0 880 674 B1, die WO 98/04881 und die WO 01/07866 A1.

Durch den Einsatz jeweils mehrerer Kameras oder aber einer Kamera und eines zusätzlichen Laser-Entfernungsmessers (wie gelehrt in der EP 0 880 674 B1) bzw. einer relativ aufwendigen Markierungseinrichtung (wie gelehrt in der WO 98/04881) sind diese neueren Entwicklungen nicht weniger aufwendig als praktisch etablierte Systeme und daher nicht geeignet, den Einsatzbereich der erwähnten Systeme auf kostensensitive Applikationen zu erweitern.

In industriellen Applikationen oder bei der sogenannten "Motionanalyse" kommen auch Positionssysteme mit einer einzelnen Kamera zum Einsatz. Beispiele hierfür sind das Produkt TraceCam-Single der Fa. Icon 3D Systems oder das System Solo der Fa. Metronor. Bei beiden Systemen ist eine Kodierung bzw. Individualisierung der verschiedenen Markerelemente einer mit der Kamera zusammenwirkenden Markierungseinrichtung sowie eine "on-the-job"-Kalibrierung (in Bezug auf weitere Kameras) erforderlich.

Es ist hierbei also sowohl die Gestaltung der Markierungseinrichtung als auch die Handhabung relativ aufwendig. Zudem benötigen flächige kodierte Markerelemente eine relativ große Anbringungsfläche, und eine gute Erkennung durch die zugeordnete Kamera ist nur in einem relativ eingeschränkten Winkelbereich möglich. Eine elektronische Kodierung der Markerelemente, die als solche grundsätzlich bekannt und etwa bei aktiv lichtgebenden Einrichtungen auf LED-Basis auch technisch realisierbar ist, erfordert einen hohen technischen Aufwand.

Aus der US 5,440,392 A ist ein Positionsbestimmungssystem nach dem Oberbegriff des Anspruchs 1 bekannt. Mit dem dort beschriebenen Verfahren kann eine recht verlässliche Positionsbestimmung des Markers erfolgen, es hat aber den Nachteil einer aufwändigen und relativ langsamen Auswertung, da die erfassten Bildpunkte in allen Kombinationen mit einem nicht-linearen Rückwärtsschnitt in den Objektraum transformiert werden müssen, um anschließend eine gültige Lösung des Koordinatenbestimmungs-Problems im Objektraum herauszufiltern. Dieser Algorithmus ist insgesamt sehr rechenaufwändig.

Der Erfindung liegt daher, gemäß einem ersten relativ unabhängigen Aspekt, die Aufgabe zugrunde, ein kostengünstiges Positionsbestimmungssystem der gattungsgemäßen Art anzugeben, welches gleichwohl mit der erforderlichen Genauigkeit und Zuverlässigkeit arbeitet und leicht bedienbar ist. Gemäß einem zweiten relativ unabhängigen Aspekt liegt der Erfindung die Aufgabe der Bereitstellung eines gegen bestimmte Handhabungsfehler besonders unempfindlichen Positionsbestimmungssystems zugrunde.

Diese Aufgabe wird durch ein Positionsbestimmungssystem mit den Merkmalen des Anspruchs 1 gelöst. Zweckmäßige Fortbildungen des Erfindungsgedankens sind Gegenstand der abhängigen Ansprüche.

Die Erfindung schließt, gemäß dem erwähnten ersten Aspekt, den Gedanken ein, ein System mit einer einzelnen Bildaufnahmeeinrichtung ("Mono-Kamera") und ohne zusätzliche Fühler bzw. Sensoren anzugeben und hierdurch die Systemkosten wesentlich zu senken. Gemäß dem zweiten Aspekt kann das System mehrere Bildaufnahmeeinrichtungen aufweisen, wobei deren Signalverarbeitung auf flexible Weise an etwaige Einschränkungen des Gesichtsfeldes einer Kamera oder des Verdecktseins einzelner Markerelemente des Markierungsinstrumentes (etwa infolge von Unachtsamkeiten bei der Handhabung) anpassbar ist.

Zur Erfindung gehört weiterhin der Gedanke, ein verbessertes Markierungselement (auch als "Lokator" bezeichnet) vorzusehen, das eine sichere Positionsbestimmung eines hiermit in Berührung stehenden Körperkontaktabschnittes auch mit dieser einzelnen Bildaufnahmeeinrichtung ermöglicht. Dieses neuartige Markierungsinstrument umfasst mindestens fünf im wesentlichen identische (als solche nicht notwendigerweise voneinander unterscheidbare, d.h. unkodierte) Markerelemente, wobei mindestens ein Markerelement außerhalb einer durch vier der Markerelemente aufgespannten Ebene liegt.

Schließlich ist eine zum System gehörende Datenverarbeitungseinrichtung zur Berechnung der räumlichen Lage des besagten Markierungsinstrumentes allein aus dem (monoskopischen) Bild der einzelnen Bildaufnahmeeinrichtung ausgebildet. Sie enthält ein entsprechend zugeschnittenes Auswertungsprogramm und angepasste Speicher- und Verarbeitungsmittel. Die für die hier auszuführenden Berechnungen notwendigen Grundlagen sind dem Fachmann vertraut; vgl. etwa T. Luhmann: Nahberechnungsfotogrammetrie - Grundlagen, Methoden und Anwendungen, Wichmann Verlag, Heidelberg 2003.

Vereinfacht formuliert, erfolgt beim erfindungsgemäßen Positionsbestimmungssystem eine Zuordnung ("Dekodierung") der räumlichen Position der einzelnen Markerelemente durch Nutzung der Gesetze der perspektivischen Abbildung. Hierbei werden Raumgeraden im Objektraum auch im aufgenommenen Bild als Geraden abgebildet.

Über geeignete und in der Literatur bekannte Filterverfahren werden im Bild die Bildpunkte gefunden. Diese werden in einer Liste nach ihren Bildkoordinaten sortiert abgelegt und dann einer logischen Prüfung unterzogen. Mit Hilfe der Lokatorgröße kann die maximale Ausdehnung des Punktfeldes im Bild und somit ein Indexbereich in der Bildpunktliste festgelegt werden. Durch dieses Verfahren wird die Ausdehnung des Lokators in der zum Bild parallelen Objektebene abgefragt. Mit Hilfe der Punktgröße (ein entfernter Punkt wird kleiner) wird überprüft, ob alle Punke in ähnlicher Entfernung liegen.

Aufgrund der vorgeschriebenen Lokatorgeometrie werden sodann zwei Geraden (4 Punkte) gesucht, deren Schnittpunkte innerhalb der Konfiguration der Bildpunkte liegen. Sind alle Vorbedingungen enthalten, wird mit Hilfe der Doppelverhältnisse eine grobe Überprüfung des Punktfeldes durchgeführt. Mit Hilfe einer "Nummerierungsrichtung" werden die möglichen Lösungen auf vier reduziert. Im Positivfall werden diese vier möglichen Lösungen in die vier ebenen Punkte des Lokators mit Hilfe der Projektivtransformation transformiert. Dies ergibt prinzipiell immer eine Lösung. Nun wird innerhalb eines definierten Gültigkeitsbereiches (Punktgröße und Abstand) nach einem fünften Punkt (hier Hochpunkt) gesucht. Ist einer vorhanden, kann dann die Gültigkeit der Lösung überprüft werden.

Ungeachtet des gegenüber bekannten Lokatoren etwas aufwendigeren Markierungsinstrumentes und der speziellen Ausgestaltung der Datenverarbeitungseinrichtung erbringt die Gesamtlösung erhebliche Kostenvorteile, die die Erschließung von neuen Teilmärkten erlaubt, für die die etablierten Systeme zu teuer waren.

In einer in vorteilhafter Weise an häufig in der Chirurgie gebräuchliche Systeme angelehnten Ausführung hat das Markierungsinstrument als Markerelemente sogenannte Retrokugeln als passiv lichtgebende Einrichtungen, die die Strahlung einer Licht- bzw. IR-Quelle zur Bildaufnahmeeinrichtung annähernd diffus reflektieren. Bevorzugt haben die Markerelemente bezüglich eines Aufnahmeabstandes der Bildaufnahmeeinrichtung vernachlässigbar kleinen Durchmesser, sind also in dieser Näherung als punktförmig anzusehen. Weiter vorteilhaft ist im Positionsbestimmungssystem eine hierauf abgestimmte und Störungen durch Umgebungslicht weitgehend vermeidende Strahlungsquelle vorgesehen, die bevorzugt IR-Strahlung (oder ggf. auch sichtbares Licht in einem geeignet gewählten Frequenzbereich) emittiert.

Alternativ zur vorgenannten Ausführung sind die Markerelemente durch aktiv lichtgebende Einrichtungen, insbesondere LEDs, insbesondere mit bezüglich eines Aufnahmeabstandes der Bildaufnahmeeinrichtung vernachlässigbar kleiner Lichtemissionsfläche, gebildet. Weiter von Vorteil ist es, wenn die lichtgebenden Einrichtungen eine, insbesondere sphärisch, gekrümmte Lichtemissionsfläche aufweisen und dadurch einen großen Erfassungs-Winkelbereich haben.

Weiter alternativ können statt der oben erwähnten Markierungsinstrumente mit näherungsweise punktförmigen Markerelementen auch solche mit flächigen Markerelementen - also Reflexionsflächen oder Leuchtflächen - eingesetzt werden, wobei jedoch keine Individualisierung der Markerflächen durch spezifische Umrissform und/oder Größe vorzusehen ist.

In einer weiter bevorzugten Ausführung ist vorgesehen, dass das Markierungsinstrument einen Grundkörper aufweist, welcher derart geformt ist, dass mindestens ein Markerelement außerhalb einer durch vier der Markerelemente aufgespannten Ebene liegt. Geeignete konkrete Formen des Grundkörpers sind eine Modifikation der als solche bekannten Dreibein-Form oder eine asymmetrische Kreuz-Form mit im oder nahe dem Zentrum des Kreuzes zusätzlich aufgesetztem Halter für das fünfte Markerelement. Die Markerelemente sitzen jeweils bevorzugt an den oder nahe der Enden der Grundkörper-Zweige.

Eine hinsichtlich der Datenverarbeitungseinrichtung zweckmäßige Systemausführung sieht vor, dass in einem Markergeoemetrie-Speicher die Geometrie des Markierungsinstrumentes und in einem Programmspeicher ein kombinierter Algorithmus zur Bestimmung einer geometrischen Relation zwischen den aktuellen Raumpunkten der Markerelemente und zur anschließenden Berechnung eines Rückwärtsschnittes abgelegt ist. Eine Berechnungsstufe der Verarbeitungseinrichtung steht mit beiden Speichern in Datenverbindung und arbeitet diesen kombinierten Algorithmus derart ab, dass die globale Lage des Punktfeldes der Markerelemente errechnet wird. Mit der bekannten Geometrie des Markerkörpers lässt sich hieraus, in an sich bekannter Weise, die benötigte Lage des Körperkontaktabschnittes des zu vermessenden Körpers gewinnen.

Zur Ausführung des Systems gemäß dem oben erwähnten zweiten Aspekt der Aufgabe und vorgeschlagenen Lösung umfasst das System eine Bildverarbeitungs-Steuereinheit, mindestens zwei Bildaufnahmeeinrichtungen und eine Bildverarbeitungs-Steuereinrichtung, welche in Reaktion auf Einschränkungen des Gesichtsfeldes einer der Bildaufnahmeeinrichtungen eine Verarbeitung von deren Bild unterbindet und die Verarbeitung des Bildes einer anderen Bildaufnahmeeinrichtung aktiviert oder die bei Erfassung von jeweils mindestens drei Markerelementen des Markierungsinstrumentes in den Bildern mindestens zweier der Bildaufnahmeeinrichtungen eine Positionsbestimmung des Markierungsinstrumentes aufgrund beider Bilder nach einem konventionellen Positionsbestimmungsverfahren, insbesondere unter Nutzung des Vorwärtsschnittes, steuert.

In dieser Systemkonfiguration ist also eine flexible, automatische Selektion verschiedener Verarbeitungsmöglichkeiten mehrerer verfügbarer Bilder der Bildaufnahmeeinrichtungen in Abhängigkeit von der problembezogenen Aussagekraft der Bilder möglich. Ist etwa durch einen Bediener die Sicht einer Kamera auf das Markierungsinstrument verdeckt, wird die Verarbeitung auf das einwandfreie Bild einer anderen Kamera umgeschaltet. Ähnlich wird, wenn aufgrund einer ungünstigen Positionierung des Markierungsinstrumentes einzelne Markerelemente im Gesichtsfeld einer oder mehrerer Kameras verdeckt sind, eine kombinierte Verarbeitung der parallel vorliegenden Bilder mehrerer Kameras, insbesondere in Art der konventionellen Stereobild-Positionsbestimmungsverfahren, angesteuert. Hierzu reicht es bekanntlich aus, jeweils 3-4 Markerelemente in mindestens zwei Bildern erfasst zu haben.

Bevorzugt weist die Bildaufnahmeeinrichtung ein hochauflösendes, kalibriertes fotoelektrisches Bildaufnahmeelement, insbesondere vom CCD- oder CMOS-Typ, auf. Es ist wichtig, dass das Bildaufnahmeelement reproduzierbar hochgenau arbeitet, insbesondere im Betrieb eine temperaturstabile Messgenauigkeit von ≤ 0,3 µm, bevorzugt ≤ 0,1 µm, aufweist. Dies ist durch geeignete konstruktive Maßnahmen sicherzustellen, die indes nicht Gegenstand der vorliegenden Erfindung sind.

Das Markierungsinstrument als solches ist für die überwiegende Zahl der Anwendungen bevorzugt als manuell zu führender sogenannter Handtaster ausgeführt, alternativ ist aber auch eine spezielle konstruktive Ausführung als sogenannter "ortsfester Lokator" möglich, deren konstruktive Merkmale als solche ebenfalls bekannt sind.

## Patentansprüche

1. Positionsbestimmungssystem zur Bestimmung der Lage eines Körpers oder Körperabschnittes im Raum, mit
- einem Markierungsinstrument, welches mindestens fünf strahlungserzeugende oder -reflektierende, unkodierte Markerelemente in im wesentlichen räumlich starrer Geometrie zueinander und zu einem Körperkontaktabschnitt des Instruments aufweist, zum mindestens temporären In-Kontakt-Bringen mit dem Körper oder Körperabschnitt,
- einer ortsfesten Bildaufnahmeeinrichtung zur Aufnahme eines monoskopischen Bildes der Markerelement-Konfiguration und
- einer eingangsseitig mit der Bildaufnahmeeinrichtung verbundenen Datenverarbeitungseinrichtung zur Verarbeitung des Bildes oder der Bilder des Markierungsinstrumentes und zur Bestimmung einer Raumposition seines Körper-Kontaktabschnittes aufgrund einer Vermessung des Bildes bzw. der Bilder, wobei die Datenverarbeitungseinrichtung zur Identifizierung der einzelnen Markerelemente aufgrund von deren Geometrie am Markierungsinstrument und zur hierauf aufbauenden fotogrammetischen Berechnung der räumlichen Lage des Markierungsinstrumentes allein aus dem monoskopischen Bild einer einzelnen Bildaufnahmeeinrichtung ausgebildet ist,
**dadurch gekennzeichnet, dass**
das Markierungsinstrument einen Grundkörper aufweist, welcher derart geformt ist, dass mindestens ein Markerelement außerhalb einer durch vier der Markerelemente aufgespannten Ebene liegt.

2. Positionsbestimmungssystem nach Anspruch 1, **dadurch gekennzeichnet,**
**dass**
die Markerelemente durch Retrokugeln, insbesondere mit bezüglich eines Aufnahmeabstandes der Bildaufnahmeeinrichtung vernachlässigbarer kleinem Durchmesser, gebildet sind und das Positionsbestimmungssystem insbesondere eine hierauf abgestimmte Lichtquelle aufweist.

3. Positionsbestimmungssystem nach Anspruch 1, **dadurch gekennzeichnet,**
**dass**
die Markerelemente durch aktiv lichtgebende Einrichtungen, insbesondere LEDs, insbesondere mit bezüglich eines Aufnahmeabstandes der Bildaufnahmeeinrichtung vernachlässigbar kleiner Lichtemissionsfläche, gebildet sind.

4. Positionsbestimmungssystem nach Anspruch 3, **dadurch gekennzeichnet,**
**dass**
die lichtgebenden Einrichtungen eine, insbesondere sphärisch, gekrümmte Lichtemissionsfläche aufweisen.

5. Positionsbestimmungssystem nach einer der vorangehenden Ansprüche,
**dadurch gekennzeichnet, dass**
der Grundkörper fünf oder mehr Zweige bzw. Fortsätze aufweist und die Markerelemente an oder nahe deren freien Enden platziert sind.

6. Positionsbestimmungssystem nach einem der vorangehenden Ansprüche,
**dadurch gekennzeichnet, dass**
in einem Markergeometrie-Speicher der Datenverarbeitungseinrichtung die Geometrie des Markierungsinstrumentes und in einem Programmspeicher ein kombinierter Algorithmus zur Bestimmung einer geometrischen Relation zwischen den Punkten der Markerelemente, und zur anschließenden Berechnung eines Rückwärtsschnittes abgelegt ist und eine Berechnungsstufe mit beiden Speichern in Datenverbindung steht.

7. Positionsbestimmungssystem nach einem der vorangehenden Ansprüche,
**gekennzeichnet durch**
eine einzelne Bildaufnahmeeinrichtung.

8. Positionsbestimmungssystem nach einem der Ansprüche 1 bis 6, **gekennzeichnet durch**
mindestens zwei Bildaufnahmeeinrichtungen und
eine Bildverarbeitungs-Steuereinrichtung, welche in Reaktion auf Einschränkungen des Gesichtsfeldes einer der Bildaufnahmeeinrichtungen eine Verarbeitung von deren Bild unterbindet und die Verarbeitung des Bildes einer anderen Bildaufnahmeeinrichtung aktiviert oder die bei Erfassung von jeweils mindestens drei Markerelementen des Markierungsinstrumentes in den Bildern mindestens zweier der Bildaufnahmeeinrichtungen eine Positionsbestimmung des Markierungsinstrumentes aufgrund beider Bilder nach einem konventionellen Positionsbestimmungsverfahren, insbesondere unter Nutzung des Vorwärtsschnittes, steuert.

9. Positionsbestimmungssystem nach einem der vorangehenden Ansprüche,
**dadurch gekennzeichnet, dass**
die Bildaufnahmeeinrichtung ein hochauflösendes, kalibriertes fotoelektrisches Bildaufnahmeelement, insbesondere vom CCD- oder CMOS-Typ, aufweist.

10. Positionsbestimmungssystem nach Anspruch 9, **dadurch gekennzeichnet,**
**dass**
das Bildaufnahmeelement im Betrieb eine temperaturstabile Messgenauigkeit von ≤ 0,1 µm aufweist.

11. Positionsbestimmungssystem nach einem der vorangehenden Ansprüche,
**dadurch gekennzeichnet, dass**
das Markierungsinstrument als Handtaster ausgeführt ist.
